# EUROPEAN PATENT APPLICATION

(11) **EP 3 434 692 A1**
(43) Date of publication of application: **30.01.2019**
(21) Application number: 17315006.1
(22) Date of filing: 24.07.2017
(51) Int. Cl.: C07K 16/28, C07D 403/04

(54) **COMPOUNDS WHICH SPECIFICALLY BINDS TO CD38 FOR USE IN THE TREATMENT OF NEURODEGENERATIVE DISEASES**

(71) Applicant: ENCEFA, 94270 Le Kremlin-Bicêtre (FR)
(72) Inventor: Toulorge, Damien, 94270 Le Kremlin-Bicêtre (FR); Guerreiro DA Silva, Serge, 94140 Alforville (FR); Bressac, Laurence, 92120 Montrouge (FR)
(74) Representative: Belbeoc'h, Stéphanie

(57) **Abstract**

The present invention relates to a compound, which specifically binds to CD38, for use as a medicament in the prevention and/or treatment of a neurodegenerative disease, by the opening of NAADP receptors Two Pore Channels TPC1 and/or TPC2, wherein said compound activates the opening of NAADP receptors Two Pore Channels TPC1 and/or TPC2.

## Description

The present invention relates to the field of prevention and/or treatment of neurodegenerative diseases.

In particular, the invention relates to a compound, useful for the prevention and/or treatment of neurodegenerative diseases, said compound specifically binding to CD38 and activating the opening of NAADP receptors Two Pore Channels TPC1 and/or TPC2.

Neurodegenerative disease is an umbrella term for a range of conditions which primarily affect the neurons in the human brain and spinal cord. Neurons are the building blocks of the nervous system which includes the brain and spinal cord. Neurons normally don't reproduce or replace themselves, so when they become damaged or die they cannot be replaced by the body. Examples of neurodegenerative diseases include Parkinson's, Alzheimer's diseases, or amyotrophic lateral sclerosis which result in progressive degeneration and / or death of nerve cells. Symptoms of such diseases relate to impairments of movement (called ataxias), or mental functioning (called dementias).

The process of neurodegeneration is not well-understood so the diseases that stem from it have, as yet, no cures. Medications, brain surgery, and multidisciplinary management are the therapeutic strategies used to provide relief from the symptoms in some patients. In the case of Parkinson's disease, levodopa has been the most widely used drug to reduce motor symptoms, however, its effect is temporary and invalidating side-effects occur with time. In Alzheimer's disease, drugs such as acetylcholine esterase inhibitors have also positive effects on the patients' symptoms but their efficacy is mainly observed, at an early stage of the disease.

Strategies targeting the inhibition of degeneration of neurons are now explored. Agents currently under investigation include in particular calcium channel blockers (isradipine) or antagonists of the NAADP receptor such as those described in WO2016024246. However, none of these agents are clinically validated.

Besides, known agents target only one type of neurodegenerative mechanism (e.g. excitotoxicity, oxidative stress or energy deficit due to mitochondrial abnormalities...).

Consequently, there remains a significant need in the art for new and improved treatment for all type of patients affected by a neurodegenerative disorder, using agent able to target different types of neurodegenerative mechanisms.

CD38 is a 45kDa type II transmembrane glycoprotein with a long C-terminal extracellular domain and a short N-terminal cytoplasmic domain. CD38 have both a receptor-mediated function and an enzyme-mediated function. As a receptor, CD38 can interact with its ligand CD31. As an ectoenzyme, CD38 is a multi-functional protein that catalyzes several reactions: (i) the conversion of nicotinamide adenine dinucleotide (NAD⁺) into adenosine diphosphate-ribose (ADPR); (ii) the conversion of NAD⁺ into cyclic ADPR (cADPR); (iii) the hydrolysis of cADPR into ADPR; (iv) in the presence of nicotinic acid, the conversion of NADP⁺, the phosphorylated equivalent of NAD⁺, into nicotinic acid adenine dinucleotide phosphate (NAADP, NAADP synthase activity); (v) the conversion of NAADP into ADPR phosphate (ADPRP, NAADP hydrolase activity) (Malavasi et al., Physiol Rev, 2008, 88: 841-886).

CD38 has been extensively studied in cancer research since this protein is upregulated in many hematopoietic malignancies and its expression is correlated with disease progression. Several antibodies were developed and used first as a research tool (clone 90, AT1, AT13/5, AT2, HB-7, IB4, IB6, OKT-10, SUN-4B7, T16) and then as therapeutics against cancer (including SAR650984 currently under phase III clinical trial (NCT02990338), MOR202 currently under phase II (NCT01421186) and Ab79 under preclinical development). Recently, daratumumab was approved for multiple myeloma patients who previously received three or more prior lines of therapy.

Since CD38 is the main cellular NADase (Chini, Curr Pharm Des, 2009, 15(1):57-63), and that NAD⁺ or its precursors were shown to be neuroprotective in a large number of studies (Harlan et al., J Biol Chem, 2016, 291(20):10836-46; Lu et al., Exp Ther Mad, 2014, 8(3):943-50; Gong et al., Neurobiol Aging, 2013, 34(6):1581-8; patent application US20120328526), CD38 was proposed as a new therapeutic target for the treatment of acute or chronic neurodegenerative diseases (Kristian et al., J Neurosci Res, 2011, 89(12):1946-55). Indeed, several studies using CD38 knock-out (KO) mice demonstrated that these animals showed significant protection against ischemic brain damage (Long et al., Neurochem Res, 2017, 42(1):283-93; patent application US20120328526) as well as reduced Aβ production and neuroinflammation when crossed with Alzheimer's disease experimental mouse model APPswePS1ΔE9 (Blacher et al., Ann Neurol, 2015, 78(1):88-103) due to increased NAD⁺ levels, suggesting that inhibition of CD38 enzymatic activity might be neuroprotective by increasing NAD⁺ levels.

Unexpectedly, the inventors have demonstrated that compounds, which specifically bind to CD38 and activate the opening of NAADP receptors Two Pore Channels TPC1 and/or TPC2 are able to efficiently prevent and protect neurons from degeneration by increasing cytosolic calcium levels.

These results are surprising since contrary to what has been suggested in the state of the art, compounds, which specifically bind to CD38, were neuroprotective not by increasing NAD⁺ levels as suggested in the literature, but by activating NAADP receptors TPCs, which are only very weakly activated following NAD⁺ treatment. This is in total contradiction with previously cited studies using CD38 KO mice (Long et al., Neurochem Res, 2017, 42(1):283-93; Blacher et al., Ann Neurol, 2015, 78(1):88-103; patent application US20120328526) ; with a publication of Hockey et al., (J Cell Sci, 2015, 128(2):232-8) who showed that lysosomal abnormalities in fibroblasts from Parkinson's disease patients (i) were corrected by NAADP receptors' inhibition and (ii) were exaggerated by NAADP; as well as with a study published by Fernandez et al., (Autophagy, 2016, 12(9):1487-506) who showed that the inhibition of NAADP receptors reduces cytoplasmic iron levels, which are elevated in various neurodegenerative diseases.

This is also in total contradiction with current investigations aiming to develop agents able to inhibit the degeneration of neurons but by blocking calcium channel (e.g. isradipine) or by antagonizing NAADP receptors (e.g. such as those described in WO2016024246), thus reducing cytoplasmic calcium levels.

Thus, in one aspect, the invention relates to a compound, which specifically binds to CD38, particularly to human CD38, for use as a medicament in the prevention and/or treatment of a neurodegenerative disease, by the opening of NAADP receptors Two Pore Channels TPC1 and/or TPC2, wherein said compound activates the opening of NAADP receptors Two Pore Channels TPC1 and/or TPC2.

The use of a compound, which specifically binds to CD38 and activates the opening of NAADP receptors Two Pore Channels TPC1 and/or TPC2, as a medicament has in particular the following advantages:
- It efficiently prevents directly and indirectly different types of neurons from different types of degeneration:
   - the neuroprotection is not specific to a particular type of neuron. On the contrary, it is able to efficiently protect *in vitro* different types of neurons including cortical neurons comprising glutamatergic and GABAergic cells, as well as dopaminergic neurons from midbrain cultures;
   - It is able to directly protect neurons from different types of degeneration mechanisms including excitotoxicity, oxidative stress, energy deficit due to mitochondrial complex I inhibition, electrical activity deficit, as well as neurotrophic factor deprivation;
   - It is able to indirectly protect neurons from degeneration, having an anti-inflammatory effect by limiting the number of microglial cells.
- It is useful for all type of patients affected by a neurodegenerative disorder considering that neurodegenerative disorders involve different types of neurons and neurodegenerative mechanisms;
- It has reduced side effects due to the specificity of compound;
- It is efficient at every stage of neurodegenerative diseases;
- Its effect is not temporary;
- It is highly efficient using low doses compared to the other tested agents (e.g. HB7 antibody totally prevented dopaminergic neurons cell death at a concentration of 7.5 nM, whereas NAD⁺ exerted its neuroprotective effect at a 3 mM concentration).

Unless defined otherwise, all technical and scientific terms used herein have the same meaning as commonly understood to one skilled in the relevant art.

For convenience, the meaning of certain terms and phrases employed in the specification, examples and claims are provided.

As used herein, the term "compound, which specifically binds to CD38" relates to any molecule suitable for pharmaceutical uses, which specifically binds to CD38. It encompasses antibodies, antigen-binding fragments thereof, antigen-binding antibody mimetics, small organic molecules, oligonucleotides and recombinant proteins.

In an embodiment, the compound according to the invention is selected from the group consisting of:
- an antibody, an antigen-binding fragment thereof, an antigen-binding antibody mimetic; and
- a small organic molecule.

As used herein, the term "antibody" comprises polyclonal antibodies, monoclonal antibodies, recombinant, bispecific, multispecific or modified antibodies.

As used herein, a "monoclonal antibody" is intended to refer to a preparation of antibody molecules, antibodies which share a common heavy chain and common light chain amino acid sequence, in contrast with "polyclonal" antibody preparations which contain a mixture of antibodies of different amino acid sequence. Monoclonal antibodies can be generated by several known technologies like phage, bacteria, yeast or ribosomal display, as well as by classical methods exemplified by hybridoma-derived antibodies. Thus, the term "monoclonal" is used to refer to all antibodies derived from one nucleic acid clone.

The antibodies of the present invention include recombinant antibodies. As used herein, the term "recombinant antibody" refers to antibodies which are produced, expressed, generated or isolated by recombinant means, such as antibodies which are expressed using a recombinant expression vector transfected into a host cell; antibodies isolated from a recombinant combinatorial antibody library; antibodies isolated from an animal (e.g. a mouse) which is transgenic due to human immunoglobulin genes; or antibodies which are produced, expressed, generated or isolated in any other way in which particular immunoglobulin gene sequences (such as human immunoglobulin gene sequences) are assembled with other DNA sequences. Recombinant antibodies include, for example, chimeric and humanized antibodies.

As used herein, a "chimeric antibody" refers to an antibody in which the sequence of the variable domain derived from the germline of a mammalian species, such as a mouse, have been grafted onto the sequence of the constant domain derived from the germline of another mammalian species, such as a human.

As used herein, a "humanized antibody" refers to an antibody in which CDR sequences derived from the germline of another mammalian species, such as a mouse, have been grafted onto human framework sequences.

In an embodiment, the invention relates to an anti-CD38 antibody or antigen-binding fragment thereof as defined above that is a humanized monoclonal antibody.

As used herein, an "antigen-binding fragment of an antibody" means a part of an antibody, *i.e.* a molecule corresponding to a portion of the structure of the antibody of the invention, that exhibits antigen-binding capacity for CD38, possibly in its native form; such fragment especially exhibits the same or substantially the same antigen-binding specificity for said antigen compared to the antigen-binding specificity of the corresponding four-chain antibody. Advantageously, the antigen-binding fragments have a similar binding affinity as the corresponding 4-chain antibodies. However, antigen-binding fragments that have a reduced antigen-binding affinity with respect to corresponding 4-chain antibodies are also encompassed within the invention. The antigen-binding capacity can be determined by measuring the affinity between the antibody and the target fragment. These antigen-binding fragments may also be designated as "functional fragments" of antibodies.

Antigen-binding fragments of antibodies are fragments which comprise their hypervariable domains designated CDRs (Complementary Determining Regions) or part(s) thereof encompassing the recognition site for the antigen, *i.e.* CD38, thereby defining antigen recognition specificity.

Each Light and Heavy chain variable domains (respectively VL and VH) of a four-chain immunoglobulin has three CDRs, designated VL-CDR1 (or LCDR1), VL-CDR2 (or LCDR2), VL-CDR3 (or LCDR3) and VH-CDR1 (or HCDR1), VH-CDR2 (or HCDR2), VH-CDR3 (or HCDR3), respectively.

The skilled person is able to determine the location of the various regions/domains of antibodies by reference to the standard definitions in this respect set forth, including a reference numbering system, a reference to the numbering system of KABAT or by application of the IMGT "collier de perle" algorithm. In this respect, for the definition of the sequences of the invention, it is noted that the delimitation of the regions/domains may vary from one reference system to another. Accordingly, the regions/domains as defined in the present invention encompass sequences showing variations in length or localization of the concerned sequences within the full-length sequence of the variable domains of the antibodies, of approximately +/- 10%.

Based on the structure of four-chain immunoglobulins, antigen-binding fragments can thus be defined by comparison with sequences of antibodies in the available databases and prior art, and especially by comparison of the location of the functional domains in these sequences, noting that the positions of the framework and constant domains are well defined for various classes of antibodies, especially for IgGs, in particular for mammalian IgGs. Such comparison also involves data relating to 3-dimensional structures of antibodies.

For illustration purpose of specific embodiments of the invention, antigen binding fragments of an antibody that contain the variable domains comprising the CDRs of said antibody encompass Fv, dsFv, scFv, Fab, Fab', F(ab')2, and single-domain antibody. Fv fragments consist of the VL and VH domains of an antibody associated together by hydrophobic interactions; in dsFv fragments, the VH:VL heterodimer is stabilised by a disulphide bond; in scFv fragments, the VL and VH domains are connected to one another via a flexible peptide linker thus forming a single-chain protein. Fab fragments are monomeric fragments obtainable by papain digestion of an antibody; they comprise the entire L chain, and a VH-CH1 fragment of the H chain, bound together through a disulfide bond. The F(ab')2 fragment can be produced by pepsin digestion of an antibody below the hinge disulfide; it comprises two Fab' fragments, and additionally a portion of the hinge region of the immunoglobulin molecule. The Fab' fragments are obtainable from F(ab')2 fragments by cutting a disulfide bond in the hinge region. F(ab')2 fragments are divalent, i.e. they comprise two antigen binding sites, like the native immunoglobulin molecule; on the other hand, Fv (a VHVL dimmer constituting the variable part of Fab), dsFv, scFv, Fab, and Fab' fragments are monovalent, i.e. they comprise a single antigen-binding site. These basic antigen-binding fragments of the invention can be combined together to obtain multivalent antigen-binding fragments, such as diabodies, tribodies or tetrabodies. These multivalent antigen-binding fragments are also part of the present invention.

As used herein, the term "bispecific" antibodies refers to antibodies that recognize two different antigens by virtue of possessing at least one region (e.g. derived from a variable region of a first antibody) that is specific for a first antigen, and at least a second region (e.g. derived from a variable region of a second antibody) that is specific for a second antigen. A bispecific antibody specifically binds to two target antigens and is thus one type of multispecific antibody. Multispecific antibodies, which recognize two or more different antigens, can be produced by recombinant DNA methods or include, but are not limited to, antibodies produced chemically by any convenient method. Bispecific antibodies include all antibodies or conjugates of antibodies, or polymeric forms of antibodies which are capable of recognizing two different antigens. Bispecific antibodies include antibodies that have been reduced and reformed so as to retain their bivalent characteristics and to antibodies that have been chemically coupled so that they can have several antigen recognition sites for each antigen such as BiME (Bispecific Macrophage Enhancing antibodies), BiTE (bispecific T cell engager), DART (Dual affinity retargeting); DNL (dock-and-lock), DVD-Ig (dual variable domain immunoglobulins), HAS (human serum albumin), kih (knobs into holes).

Accordingly, bispecific antibodies of the invention specifically bind to CD38 and a second antigen and activate the opening of NAADP receptors Two Pore Channels TPC1 and/or TPC2.

In an embodiment, the invention also encompasses an anti-CD38 antibody or antigen-binding fragment thereof or antigen-binding antibody mimetic as defined herein that is bispecific, in particular for its use as a medicament in the prevention and/or treatment of a neurodegenerative disease, by the opening of NAADP receptors Two Pore Channels TPC1 and/or TPC2.

Several researches to develop therapeutic antibodies had led to engineer the Fc regions to optimize antibody properties allowing the generation of molecules that are better suited to the pharmacology activity required of them. The Fc region of an antibody mediates its serum half-life and effector functions, such as complement-dependent cytotoxicity (CDC), antibody-dependent cellular cytotoxicity (ADCC) and antibody-dependent cell phagocytosis (ADCP). Several mutations located at the interface between the CH2 and CH3 domains, such as T250Q/M428L and M252Y/S254T/T256E + H433K/N434F, have been shown to increase the binding affinity to FcRn and the half-life of IgG1 *in vivo.* However, there is not always a direct relationship between increased FcRn binding and improved half-life. One approach to improve the efficacy of a therapeutic antibody is to increase its serum persistence, thereby allowing higher circulating levels, less frequent administration and reduced doses. Engineering Fc regions may be desired to either reduce or increase the effector function of the antibody. For antibodies that target cell-surface molecules, especially those on immune cells or neurons, abrogating effector functions is required. Conversely, for antibodies intended for oncology use, increasing effector functions may improve the therapeutic activity. The four human IgG isotypes bind the activating Fcγ receptors (FcγRI, FcyRlla, FcγIIIa), the inhibitory FcyRllb receptor, and the first component of complement (C1q) with different affinities, yielding very different effector functions. Binding of IgG to the FcyRs or C1q depends on residues located in the hinge region and the CH2 domain. Two regions of the CH2 domain are critical for FcyRs and C1q binding, and have unique sequences in IgG2 and IgG4.

As used herein, a "modified antibody" corresponds to a molecule comprising an antibody or an antigen-binding fragment thereof, wherein said antibody or fragment thereof is associated with a functionally different molecule. A modified antibody of the invention may be either a fusion chimeric protein or a conjugate resulting from any suitable form of attachment including covalent attachment, grafting, chemical bonding with a chemical or biological group or with a molecule, such as a PEG polymer or another protective group or molecule suitable for protection against proteases cleavage *in vivo,* for improvement of stability and/or half-life of the antibody or functional fragment. With similar techniques, especially by chemical coupling or grafting, a modified antibody can be prepared with a biologically active molecule, said active molecule being for example chosen among toxins, in particular Pseudomonas exotoxin A, the A-chain of plant toxin ricin or saporin toxin, especially a therapeutic active ingredient, a vector (including especially a protein vector) suitable for targeting the antibody or functional fragment to specific cells or tissues of the human body, or it may be associated with a label or with a linker, especially when fragments of the antibody are used. PEGylation of the antibody or functional fragments thereof is a particular interesting embodiment as it improves the delivery conditions of the active substance to the host, especially for a therapeutic application. PEGylation can be site specific to prevent interference with the recognition sites of the antibodies or functional fragments, and can be performed with high molecular weight PEG. PEGylation can be achieved through free cysteine residues present in the sequence of the antibody or functional fragment or through added free Cysteine residues in the amino sequence of the antibody or functional fragment.

In an embodiment, the invention also encompasses an anti-CD38 antibody or antigen-binding fragment thereof or antigen-binding antibody mimetic as defined herein that is modified, in particular for its use as a medicament in the prevention and/or treatment of a neurodegenerative disease, by the opening of NAADP receptors Two Pore Channels TPC1 and/or TPC2.

As used herein the term "antigen-binding antibody mimetic" refers to artificial proteins, peptides and any chemical compounds with the capacity to bind antigens mimicking that of antibodies.

Such mimetics comprise oligonucleotide aptamers. As used herein, the term "oligonucleotide" relates to a short molecule of nucleotides including DNA, RNA but also modified nucleotides (such as nucleotides comprising at least one chemical modification). In particular, said oligonucleotide consists of less than 200 nucleotides, more particularly of less than 50 nucleotides. As used herein, the term "oligonucleotide aptamer" refers to short oligonucleotides that can selectively bind to small molecular ligands or protein targets with high affinity and specificity, when folded into their unique three-dimensional structures.

Such mimetics comprise affitins and anticalins as well as peptide aptamers. Affitins are artificial proteins with the ability to selectively bind antigens. They are structurally derived from the DNA binding protein Sac7d, found in *Sulfolobus acidocaldarius,* a microorganism belonging to the archaeal domain. By randomizing the amino acids on the binding surface of Sac7d, e.g. by generating variants corresponding to random substitutions of 11 residues of the binding interface of Sac7d, an affitin library may be generated and subjecting the resulting protein library to rounds of ribosome display, the affinity can be directed towards various targets, such as peptides, proteins, viruses and bacteria. Affitins are antibody mimetics and are being developed as tools in biotechnology. They have also been used as specific inhibitors for various enzymes (Krehenbrink et al., J. mol. Biol., 2008, 383(5):1058-68). The skilled person may readily develop anticalins with the required binding properties using methods know in the art, in particular as disclosed in patent application WO2008068637 and the above-cited publication, in particular the generation of phage display and/or ribosome display libraries and their screening using an antigen as dislosed herein. Anticalins are artificial proteins that are able to bind to antigens, either to proteins or to small molecules. They are antibody mimetic derived from human lipocalins which are a family of naturally binding proteins. Anticalins are about eight times smaller with a size of about 180 amino acids and a mass of about 20 kDa (Skerra, FEBS J., 2008, 275(11):2667). Anticalin phage display libraries have been generated which allow for the screening and selection, in particular of anticalins with specific binding properties. The skilled person may readily develop affitins with the required binding properties using methods known in the art, in particular as disclosed in EP patent EP1270725 B1, US patent US8536307 B2, (Schlehuber and Skerra, Biophys. Chem., 2002, 96(2-3):213-28) and the above-cited publication, in particular the generation of phage display and/or ribosome display libraries and their screening using an antigen as disclosed herein. As used herein the term "peptide aptamers » refers to small combinatorial proteins that are selected to bind to specific sites on their target molecules (antigens). Anticalins and affitins and peptide aptamers may be produced in a number of expression system comprising bacterial expression systems or by combinatorial chemistry. Thus, the invention provides affitins, anticalins, peptide aptamers and other similar antibody mimetics with the features of the antibodies described herein, in particular with regard to the specific binding to CD38 and to the activation of the opening of NAADP receptors Two Pore Channels TPC1 and/or TPC2.

All the embodiments disclosed herein for antibodies or fragments thereof are transposed *mutatis mutandis* to the antigen-binding antibody mimetics of the invention.

As used herein, the term "CD38" refers to the 45kDa type II transmembrane glycoprotein also known as T10, cyclic ADP-ribose hydrolase 1, ADPRC1 as described in Malavasi et al. (Physiological Review, 2008, 88:841-886), particularly from a mammal species, more particularly a human CD38.

Preferably, the term "human CD38" refers to the protein of amino acid sequence referenced by the NP_001766 NCBI accession number. The numbering of amino acids of human CD38 as described herein corresponds to the numbering of amino acids of the human CD38 sequence referenced by the NP_001766 NCBI accession number.

As used herein, the term "anti-CD38 antibody" refers to an antibody which specifically binds to CD38, in particular to a human CD38.

The specific binding between the compound according to the invention (in particular the antibody or antigen-binding fragment thereof or antigen-binding antibody mimetic according to the invention) and CD38 (in particular the epitope within CD38) implies that the compound according to the invention (in particular the antibody or antigen-binding fragment thereof or antigen-binding antibody mimetic according to the invention) exhibits appreciable affinity for CD38 (in particular the epitope within CD38).

When the compound is an antibody or antigen-binding fragment thereof or antigen-binding antibody mimetic, "appreciable affinity" includes binding with an affinity of about 10⁻⁸ M (KD-affinity constant) or stronger. Preferably, binding is considered specific when the binding affinity is between 10⁻⁸ M and 10⁻¹² M, optionally between 10⁻⁹ M and 10⁻¹⁰ M, in particular 10⁻¹⁰ M.

When the compound is a small organic molecule, "appreciable affinity" includes binding with an affinity of about 10⁻⁶ M (KD-affinity constant) or stronger. Preferably, binding is considered specific when the binding affinity is between 10⁻⁶ M and 10⁻¹⁰ M, optionally between 10⁻⁷ M and 10⁻¹⁰ M, in particular 10⁻⁸ M.

When the compound is an oligonucleotide, "appreciable affinity" includes binding with an affinity of about 200 nM (KD-affinity constant) or stronger. Preferably, binding is considered specific when the binding affinity is between 100 nM and 200 nM, optionally between 100 nM and 150 nM, in particular 100 nM.

Whether a binding domain specifically reacts with or binds to a target can be tested readily by, inter alia, comparing the reaction of said binding domain with a target protein or antigen with the reaction of said binding domain with proteins or antigens other than the target protein.

The affinity can be determined by various methods well known from one skilled in the art. These methods include, but are not limited to, Biacore Analysis, Blitz analysis and Scatchard plot.

In an embodiment, the anti-CD38 antibody or antigen-binding fragment thereof or antigen-binding antibody mimetic according to the invention has a KD value (affinity constant) inferior or equal to 10⁻⁸ M, preferably inferior or equal to 10⁻⁹ M for CD38, more preferably inferior or equal to 1.10⁻¹⁰ M, as may be determined by biosensor analysis, particularly by Biacore Analysis.

As used herein, the term "NAADP receptors Two Pore Channels TPC1 and/or TPC2" relates to the receptors also known as TPCN1 and TPCN2 as described in Patel et al. (Science signalling, 2015, 8(384):re7).

Preferably, the term "NAADP receptor Two Pore Channels TPC1" refers to the human TPC1 of amino acid sequence referenced by the NP_001137291.1 NCBI accession number.

Preferably, the term "NAADP receptor Two Pore Channels TPC2" refers to the human TPC2 of amino acid sequence referenced by the NP_620714.2 NCBI accession number.

As used herein, the term "activates the opening of NAADP receptors Two Pore Channels TPC1 and/or TPC2" relates to the activation of NAADP receptors Two Pore Channels TPC1 and/or TPC2 leading to their opening and the release of Ca²⁺ (as described in Patel et al. (Science signalling, 2015, 8(384):re7)) from lysosomal Ca²⁺ stores to the cytoplasm.

The capacity of a compound to activate the opening of NAADP receptors Two Pore Channels TPC1 and/or TPC2 can be tested by using an inhibitor of TPC1 and/or TPC2 activation, such as NedK (Davidson et al., Cardiovascular Research, 2015, 108, 357-366) or such as Ned-19 (Arndt et al., Molecular Biology of the Cell, 2014, 25(6):948-64; Calcraft et al., Nature, 2009, 459(7246):596-600, Naylor et al., Nature Chemical Biology, 2009, 5(4):220-6; Lee et al., Scientific Reports, 2016, 6:20282), as described in the examples below.

In particular, the neuroprotective effect of a compound according to the invention, which specifically binds to CD38 is antagonized by at least 20%, 30%, 40%, 50%, particularly at least 60% and more particularly at least 70% in the presence of NedK and/or Ned-19, as compared to a negative control in the absence of NedK and/or Ned-19 in a neuroprotective assay as described in the examples below. These neuroprotective assays include but are not limited to assays on prevention of spontaneous and progressive dopaminergic (DA) neuron death in midbrain cultures, protection of dopaminergic neurons against the mitochondrial neurotoxin MPP⁺, protection of dopaminergic neurons from neurotrophic factor deprivation, protection of cortical neurons from oxidative stress, and protection of cortical neurons from excitotoxicity, in the presence or absence of Ned-19, as described in the examples below.

The compound according to the invention, which specifically binds to CD38 and activates the opening of NAADP receptors Two Pore Channels TPC1 and/or TPC2 according to the invention has the advantage to directly and indirectly protect different type of neurons from different types of neurodegenerative mechanisms.

The compound according to the invention, which specifically binds to CD38 and activates the opening of NAADP receptors Two Pore Channels TPC1 and/or TPC2 according to the invention has at least one of the following properties, in particular the whole properties:
- it prevents spontaneous and progressive cell death of neurons, in particular of midbrain dopaminergic neurons,
- it protects dopaminergic neurons against energy deficit due to mitochondrial complex I inhibition, in particular against the mitochondrial neurotoxin MPP+,
- it protects dopaminergic neurons against neurotrophic factor deprivation, in particular GDNF deprivation,
- it protects neurons, in particular cortical neurons, from excitotoxicity, in particular against glutamate insult;
- It protects cortical neurons from oxidative stress, in particular against H₂O₂ insult, and,
- it has an anti-inflammatory effect by limiting the number of microglial cells.

In an embodiment, the compound according to the invention increases intracellular NAADP levels, in particular in neurons, by at least 10%, 20%, 30%, 40%, 50%, particularly at least 60% and more particularly at least 70%, as compared to a negative control molecule, in a NAADP level measurement assay.

Methods for determining the intracellular NAADP levels are well known in the art, such as by HPLC and Mass Spectrum analyses (see, Berridge et al., Biochemical Journal, 2002, 365:295-301).

In particular, the compound according to the invention increases intracellular NAADP levels, in particular in neurons, by inhibiting the NAADP hydrolase activity of CD38 (particularly of human CD38) or by activating the NAADP synthase activity of CD38 (particularly of human CD38).

In particular, the compound according to the invention increases intracellular NAADP levels, in particular in neurons, by inhibiting the ability of CD38 (particularly of human CD38) to degrade NAADP (in particular, into ADPRP) (by inhibiting the NAADP hydrolase activity of CD38) or by activating the ability of CD38 (particularly of human CD38) to synthetize NAADP (in particular, from NADP⁺, in the presence of nicotinic acid) (by activating the NAADP synthase activity of CD38).

The ability of a compound to inhibit the NAADP hydrolase activity of CD38 (i.e. to inhibit the ability of CD38 to degrade NAADP, in particular, into ADPRP) can be assayed using a method well known in the art, such as *in vitro* using a solution comprising at least NAADP and CD38 (from recombinant CD38 proteins, extracted cellular proteins or whole cells expressing CD38; biochemical or cell-based assay) as described in Schmid et al. (2011, FEBS letters, vol. 585, Issue 22, 3544-3548).

The ability of a compound to activate the NAADP synthase activity of CD38 (i.e. to activate the ability of CD38 to synthetize NAADP, in particular, from NADP⁺ in the presence of nicotinic acid; NAADP synthase activity) can be assayed using a method well known in the art, such as *in vitro* using a solution comprising at least NADP, nicotinic acid and CD38 (from recombinant CD38 proteins, extracted cellular proteins or whole cells expressing CD38; biochemical or cell-based assay) as described in Schmid et al. (2011, FEBS letters, vol. 585, Issue 22, 3544-3548).

In an embodiment, the compound according to the invention increases cytosolic calcium levels, in particular in neurons, by at least 10%, 20%, particularly at least 30% and more particularly at least 40%, as compared to a negative control molecule, in an intracellular calcium level measurement assay.

Methods for determining the cytosolic calcium levels are well known in the art, such as by microscopy using fluorescent calcium indicator as described in the examples below.

In one embodiment, the compound according to the invention is selected from the group consisting of an anti-CD38 antibody, an antigen-binding fragment thereof and an antigen-binding antibody mimetic.

In particular, said anti-CD38 antibody or antigen-binding fragment thereof or antigen-binding antibody mimetic according to the invention specifically binds to the peptide consisting of amino acids 70 to 300, more particularly to the peptide consisting of amino acids 220 to 285 of human CD38

The numbering of amino acids of human CD38 as described herein corresponds to the numbering of amino acids of the human CD38 sequence referenced by the NP_001766 NCBI accession number. Thus, said anti-CD38 antibody or antigen-binding fragment thereof or antigen-binding antibody mimetic according to the invention, which specifically bind to the peptide consisting of amino acids 70 to 300, more particularly to the peptide consisting of amino acids 220 to 285 of human CD38, specifically bind to the peptide consisting of amino acids at position 70 to 300, more particularly to the peptide consisting of amino acids 220 to 285 in the human CD38 sequence referenced by the NP_001766 NCBI accession number.

In one embodiment, said anti-CD38 antibody or antigen-binding fragment thereof or antigen-binding antibody mimetic according to the invention specifically binds to cysteine 254 and/or 275 of human CD38.

In one embodiment, said anti-CD38 antibody or antigen-binding fragment thereof or antigen-binding antibody mimetic according to the invention specifically binds to a region comprising the 5th (penultimate) C-terminal disulfide loop involving cysteine 254 and 275 of human CD38, in particular said region consisting of amino acids 220 to 285 of human CD38.

In one embodiment, said anti-CD38 antibody or antigen-binding fragment thereof or antigen-binding antibody mimetic according to the invention specifically binds to the 5^{th} C-terminal disulfide loop involving cysteine 254 and 275 of human CD38.

In one embodiment, said anti-CD38 antibody or antigen-binding fragment thereof or antigen-binding antibody mimetic according to the invention does not specifically bind to a region comprising the 6th C-terminal disulfide loop involving cysteine 287 and 296 of human CD38, more particularly said region consisting of amino acids 285 to 300 of human CD38.

In one embodiment, said anti-CD38 antibody or antigen-binding fragment thereof or antigen-binding antibody mimetic according to the invention does not specifically bind to the 6th C-terminal disulfide loop involving cysteine 287 and 296 of human CD38.

In one embodiment, the anti-CD38 antibody according to the invention is selected in the group consisting of HB7, clone 90, AT1, AT13/5 (as described in Table 1) and mutated, recombinant (including chimeric and humanized), bispecific and modified antibodies thereof which are able to specifically bind to CD38 and activate the opening of NAADP receptors Two Pore Channels TPC1 and/or TPC2, particularly in the group consisting of HB7, clone 90, AT1 and mutated, recombinant (including chimeric and humanized), bispecific and modified antibodies thereof which are able to specifically bind to CD38 and activate the opening of NAADP receptors Two Pore Channels TPC1 and/or TPC2, more particularly in the group consisting of HB7, clone 90 and mutated, recombinant (including chimeric and humanized), bispecific and modified antibodies thereof which are able to specifically bind to CD38 and activate the opening of NAADP receptors Two Pore Channels TPC1 and/or TPC2 and even more particularly in the group consisting of HB7 and mutated, recombinant (including chimeric and humanized), bispecific and modified antibodies thereof which are able to specifically bind to CD38 and activate the opening of NAADP receptors Two Pore Channels TPC1 and/or TPC2.

In one embodiment, the anti-human CD38 antibody of the invention is a monoclonal antibody, in particular a humanized monoclonal antibody, more particularly wherein the antibody light chain constant domain is derived from a human kappa light chain constant domain and wherein the antibody heavy chain constant domain is derived from a human IgG1, IgG2, IgG3, or IgG4 (wild type or mutated) heavy chain constant domain.

The humanized antibody or antigen-binding fragment thereof according to the invention have the advantage to be less immunogenic (or completely non-immunogenic) than murine versions in human subject to which they are administered.

As well known by one skilled in the art, the choice of IgG isotypes of the heavy chain constant domain centers on whether specific functions are required and the need for a suitable *in vivo* half-life. For example, antibodies designed for selective eradication of cancer cells typically require an active isotype that permits complement activation and effector-mediated cell killing by antibody-dependent cell-mediated cytotoxicity. Both human IgG1 and IgG3 (shorter half-life) isotypes meet these criteria, particularly human IgG1 isotype (wild type and variants). In particular, depending of the IgG isotype of the heavy chain constant domain (particularly human wild type and variants IgG1 isotype), the antibody can be cytotoxic towards cells via a CDC, ADCC and/or ADCP mechanism (Salfeld, Nature Biotechnology, 2007, 25(12):1369-72; Irani et al., Molecular Immunology, 2015, 67(2 Pt A):171-82). In fact, the fragment crystallisable (Fc) region interacts with a variety of accessory molecules to mediate indirect effector functions such as antibody-dependent cellular cytotoxicity (ADCC), antibody-dependent cellular phagocytosis (ADCP) and complement-dependent cytotoxicity (CDC).

As used herein, the term "small organic molecule" refers to a low molecular weight organic compound (up to 5000 Da, particularly up to 2000 Da, and more particularly up to 1000 Da).

In one embodiment, the small organic molecule according to the invention is selected in the group consisting of ara-2'-F-NAD⁺ (also named as ARA-F-NAD, β- ara- 2'- Deoxy- 2'-fluoro- nicotinamide adenine dinucleotide) and mutated small organic molecules thereof which are able to specifically bind to CD38 and activates the opening of NAADP receptors Two Pore Channels TPC1 and/or TPC2.

In one embodiment, the small organic molecule according to the invention inhibits the NAADP hydrolase activity of CD38 (particularly of human CD38) with an IC50 inferior or equal to 5 µM, in particular inferior or equal to 500 nM, more particularly inferior or equal to 50 nM or activates the NAADP synthase activity of CD38 (particularly of human CD38) with an EC50 inferior or equal to 5 µM, in particular inferior or equal to 500 nM, more particularly inferior or equal to 50 nM.

In one embodiment, the small organic molecule according to the invention inhibits the ability of CD38, in particular human CD38, to degrade NAADP with an IC50 inferior or equal to 5 µM, in particular inferior or equal to 500 nM, more particularly inferior or equal to 50 nM or activates the ability of CD38, in particular human CD38, to synthetize NAADP with an EC50 inferior or equal to 5 µM, in particular inferior or equal to 500 nM, more particularly inferior or equal to 50 nM.

Methods for determining the IC50 of a small organic molecule according to the invention that inhibits the ability of CD38, in particular human CD38, to degrade NAADP can be assayed *in vitro* by using a solution comprising at least NAADP, CD38 (from recombinant CD38 proteins, extracted cellular proteins or whole cells expressing CD38) in the presence or not of the compounds of interest by following NAADP degradation by HPLC as described in Schmid et al. (2011, FEBS letters, vol. 585, Issue 22, 3544-3548) or by another detection method like ELISA, or by following ADPRP formation.

Methods for determining the EC50 of a small organic molecule according to the invention that activate the ability of CD38, in particular human CD38, to synthetize NAADP can be assayed *in vitro* by using a solution comprising at least NADP, nicotinic acid, CD38 (from recombinant CD38 proteins, extracted cellular proteins or whole cells expressing CD38) in the presence or not of the compounds of interest by following NAADP synthesis by HPLC as described in Schmid et al. (2011, FEBS letters, vol. 585, Issue 22, 3544-3548) or by another detection method like ELISA, or by following NADP degradation.

In one embodiment, the small organic molecule according to the invention specifically binds to at least one, in particular at least two amino-acid(s) of human CD38 selected from the group consisting of glutamic acid 146, aspartic acid 155 and glutamic acid 226 (Graeff et al., JBC, 2006, 281(39):28951-7).

In particular, the small organic molecule according to the invention specifically binds to glutamic acid 146, aspartic acid 155 and glutamic acid 226 of human CD38.

As used herein, the term "neurodegenerative disease" refers to any condition susceptible of being improved or prevented by the protection and/or prevention of neurodegeneration.

In particular, the neurodegenerative disease is selected in the group consisting of:
- Parkinson's disease and related disorders including Parkinson's disease, Parkinson-dementia, autosomal recessive PARK2 and PARK6-linked Parkinsonism, atypical parkinsonian syndromes, such as, for example, progressive supranuclear palsy, corticobasal degeneration syndrome, Lewy bodies dementia, multiple system atrophy, Guadeloupean Parkinsonism and Lytigo-bodig disease,
- motor neuron diseases including amyotrophic lateral sclerosis, frontotemporal dementia, progressive bulbar palsy, pseudobulbar palsy, primary lateral sclerosis, progressive muscular atrophy, spinal muscular atrophy and post-polio syndrome,
- neuro-inflammatory diseases,
- Alzheimer's disease and related disorders including early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, Pick's disease, argyrophilic grain disease, posterior cortical atrophy, Wernicke-Korsakoff Syndrome,
- prion diseases including Creutzfeld-Jacob disease, lysosomal storage diseases, leukodystrophies, Huntington's Disease, Down syndrome, spinal and bulbar muscular atrophy, stroke, traumatic brain injuries, HIV-Associated Neurocognitive Disorder, Tourette Syndrome, autosomal dominant spinocerebellar ataxia, multiple sclerosis, Friedreich's Ataxia, Charcot-Marie-Tooth disease, Dentatorubral pallidoluysian atrophy, myotonic dystrophy, schizophrenia, age associated memory impairment, autism and autism spectrum disorders, attention-deficit hyperactivity disorder, chronic pain, alcohol-induced dementia, progressive non-fluent aphasia, semantic dementia, spastic paraplegia, fibromyalgia, post-Lyme disease, neuropathies, withdrawal symptoms, Alpers' disease, cerebro-oculo-facio-skeletal syndrome, Wilson's disease, Cockayne syndrome, Leigh's disease, neurodegeneration with brain iron accumulation, opsoclonus myoclonus syndrome, alpha-methylacyl-CoA racemase deficiency, Andermann syndrome, Arts syndrome, Marinesco-Sjögren syndrome, mitochondrial membrane protein-associated neurodegeneration, pantothenate kinase-associated neurodegeneration, polycystic lipomembranous osteodysplasia with sclerosing leukoencephalopathy, riboflavin transporter deficiency neuronopathy, and ataxia telangiectasia.

More particularly, the neurodegenerative disease is selected in the group consisting of:

Parkinson's disease, Lewy body dementia, multiple system atrophy, Alzheimer's disease, progressive supranuclear palsy, corticobasal degeneration syndrome, Pick's disease, frontotemporal dementia, amyotrophic lateral sclerosis, spinal and bulbar muscular atrophy, stroke, traumatic brain injuries, Huntington's disease, multiple sclerosis, Friedreich's ataxia, Charcot-Marie-Tooth disease, Creutzfeld-Jacob disease and other prion diseases, leukodistrophies, lysosomal storage diseases.

The invention also relates to a compound according to the invention, for use as a medicament, wherein said compound is administered to a non-responder patient to a treatment targeting the NAD⁺-dependent pathway and/or the cADPR-dependent pathway.

As used herein, a "medicament" is meant to encompass a composition suitable for administration to a subject or patient, such as a mammal, especially a human. In general, a "medicament" is sterile and is usually free of contaminants that are capable of eliciting an undesirable response within the subject (e.g., the compound(s) in the medicament is pharmaceutical grade). Medicaments can be designed for administration to subjects in need thereof via a number of different routes of administration including oral, buccal, rectal, parenteral, intraperitoneal, intradermal, intranasal, intrathecal, perispinal and the like. The preferred form depends on the intended mode of administration and therapeutic application. Typical preferred form are oral, injectable or infusible solutions.

In particular, the medicament according to the invention comprises as an active ingredient a compound according to the invention and a pharmaceutically acceptable carrier.

As used herein, a "pharmaceutically acceptable carrier" is meant to encompass an excipient, diluent, carrier, and adjuvant that are useful in preparing a pharmaceutical composition that are generally safe, non-toxic and neither biologically nor otherwise undesirable, and include an excipient, diluent, carrier, and adjuvant that are acceptable for veterinary use as well as human pharmaceutical use. "A pharmaceutically acceptable carrier" as used herein includes both one and more than one such excipient, diluent, carrier, and adjuvant.

The invention also relates to the use of a compound, which specifically binds to CD38 and activates the opening of NAADP receptors Two Pore Channels TPC1 and/or TPC2 for the preparation of a medicament for the prevention and/or treatment of a neurodegenerative disease.

Such compounds (antibody or antigen-binding fragment thereof or antigen-binding antibody mimetic or small organic molecule according to the invention) can be present in the medicament according to the invention in a therapeutically amount (active and non-toxic amount).

The invention also relates to a method of treatment of a neurodegenerative disease comprising the administration of a compound, which specifically binds to CD38 and activates the opening of NAADP receptors Two Pore Channels TPC1 and/or TPC2 according to the invention in a therapeutically amount to a subject in need thereof.

The term "therapeutically effective amount" refers to level or amount of agent that is aimed at, without causing significant negative or adverse side effects to the target, (1) delaying or preventing the onset of a neurodegenerative disease; (2) slowing down or stopping the progression, aggravation, or deterioration of one or more symptoms of a neurodegenerative disease; (3) bringing about ameliorations of the symptoms of a neurodegenerative disease; (4) reducing the severity or incidence of a neurodegenerative disease; or (5) curing a neurodegenerative disease.

Such therapeutically amount can be determined by one skilled in the art by routine tests including assessment of the effect of administration of said components on the pathologies and/or disorders which are sought to be prevented and/or to be treated by the administration of said pharmaceutical composition or medicament according to the invention.

For example, such tests can be implemented by analyzing both quantitative and qualitative effect of the administration of different amounts of said aforementioned compounds (antibody or antigen-binding fragment thereof or antigen-binding antibody mimetic or small organic molecules according to the invention) on a set of markers (biological and/or clinical) characteristics of said pathologies and/or of said disorders, in particular from a biological sample of a subject.

A therapeutically effective amount may be administered prior to the onset of a neurodegenerative disease, for a prophylactic or preventive action. Alternatively, or additionally, the therapeutically effective amount may be administered after the onset of a neurodegenerative disease, for a therapeutic action. In one embodiment, a therapeutically effective amount of the composition is an amount that is effective in reducing at least one symptom of a neurodegenerative disease.

Sterile medicaments for intrathecal administration can be prepared by incorporating the compound of the present invention in the required amount in the appropriate solvent, followed by sterilization by microfiltration. As solvent or vehicle, there may be used water, saline, phosphate buffered saline, dextrose, glycerol, ethanol, and the like, as well as combination thereof. In many cases, it will be preferable to include isotonic agents, such as sugars, polyalcohols, or sodium chloride in the composition. These medicaments may also contain adjuvants, in particular wetting, isotonizing, emulsifying, dispersing and stabilizing agents.

Sterile medicaments for parenteral administration may also be prepared in the form of sterile solid compositions which may be dissolved at the time of use in sterile water or any other injectable sterile medium.

The compound of the present invention may also be orally administered. As solid medicaments for oral administration, tablets, pills, powders (gelatine capsules, sachets) or granules may be used. In these medicaments, the active ingredient according to the invention is mixed with one or more inert diluents, such as starch, cellulose, sucrose, lactose or silica, under an argon stream. These medicaments may also comprise substances other than diluents, for example one or more lubricants such as magnesium stearate or talc, a coloring, a coating (sugar-coated tablet) or a glaze.

As liquid medicaments for oral administration, there may be used pharmaceutically acceptable solutions, suspensions, emulsions, syrups and elixirs containing inert diluents such as water, ethanol, glycerol, vegetable oils or paraffin oil. These medicaments may comprise substances other than diluents, for example wetting, sweetening, thickening, flavoring or stabilizing products.

The doses depend on the desired effect, the duration of the treatment and the route of administration used; they are generally between 5 mg and 1000 mg per day orally for an adult with unit doses ranging from 0.01 mg to 250 mg of active substance. In general, the doctor will determine the appropriate dosage depending on the age, weight and any other factors specific to the subject to be treated.

The invention also relates to a combination product comprising as active ingredients:
- at least one compound according to the invention; and
- at least one second therapeutic agent selected from the group consisting of neuroprotective agents, symptomatic agents, probiotics and antibodies used to neutralize aggregated or aggregation-prone proteins,
for its use as a medicament in the prevention and/or treatment of a neurodegenerative disease, by the opening of NAADP receptors Two Pore Channels TPC1 and/or TPC2, wherein said active ingredients are formulated for separate, simultaneous, or sequential administration.

The invention also relates to a combination product comprising as active ingredients:
- at least one compound according to the invention; and
- at least one second therapeutic agent selected from the group consisting of neuroprotective agents, symptomatic agents, probiotics, and antibodies used to neutralize aggregated or aggregation-prone proteins.
for its separate, simultaneous, or sequential use as a medicament in the prevention and/or treatment of a neurodegenerative disease, by the opening of NAADP receptors Two Pore Channels TPC1 and/or TPC2.

As used herein, the term "neuroprotective agent" encompasses any agent having a neuroprotective effect.

As used herein the term "probiotic" refers to any substance which stimulates the growth of and/or contains microorganisms with beneficial properties, such as those of the intestinal flora. For example, said probiotic can contain the strains of S. thermophilus, B. animalis, L. bulgaricus, Lactococcus lactis, Lactobacillus acidophilus, Lactobacillus casei, Bifidobacterium bifidum, and Lactobacillus fermentum. It has been described that the regular intake of such probiotics resulted in an improvement of the cognitive capabilities.

As used herein the term "antibody used to neutralize aggregated or aggregation-prone proteins" refers to any antibody targeting α-synudein, APP or any APP fragment (including Aβ₁₋₄₂). Tau, prion, or polyglutamine-extended proteins.

For example, said antibody used to neutralize aggregated or aggregation-prone proteins can be solanezumab or aducanumab to neutralize Aβ peptides, armanezumab to neutralize Tau, as well as PRX002 or PD0805 to neutralize α-synuclein.

As used herein the term "symptomatic agent" refers to any agent used to improve the quality of life of patients suffering from neurodegenerative diseases without altering the progression of the pathology.

In particular, when the neurodegenerative disease is ALS, said second therapeutic agent can be a neuroprotective agent selected in the group consisting of Riluzole, Edaravone (free radical scavenger) and masitinib.

In particular, when the neurodegenerative disease is an Alzheimer's disease, said second therapeutic agent can be a symptomatic agent selected in the group consisting of cholinesterase inhibitors (e.g. donepezil, galantamine, memantine, rivastigmine) and NMDA receptor antagonists (e.g. memantine).

In particular, when the neurodegenerative disease is a Parkinson's disease, said second therapeutic agent can be a symptomatic agent selected in the group consisting of Dopamine mimetics (e.g. Carbidopa, Levodopa, association of both products), Dopamine agonists (apomorphine, bromocriptine, rotigotine, pramipexole, Ropinirole), anticholinergic (benzatropine, trihexyphenidyl), MAO-B inhibitors (selegiline, rasagiline), COM-T inhibitors (entacapone, tolcapone) and amantadine, droxidopa, pimavanersin, rivastigmine.

In particular, when the neurodegenerative disease is a Huntington's disease, said second therapeutic agent can be a symptomatic agent selected in the group consisting of antipsychotic (e.g. haloperidol, chlorpromazine, risperidone, quetiapine, olanzapine), antidepressant (e.g. citalopram, fluoxetine, olanzapine), mood-stabilizing drugs (e.g. valproate, carbamazepine, lamotrigine) and tetrabenazine, amantadine, levetiracetam, clonazepam.

In particular, when the neurodegenerative disease is SMA, said second therapeutic agent can be nusinersen.

In particular, when the neurodegenerative disease is a Friedreich ataxia, said second therapeutic agent can be a neuroprotective agent selected in the group consisting of 5-hydroxytryptophan, Coenzyme Q, Idebenone.

In particular, when the neurodegenerative disease is a Multiple sclerosis, said second therapeutic agent can be an anti-inflammatory or neuroprotective agent selected in the group consisting of interferon beta-1a, peginterferon beta-1a, interferon beta-1b, glatiramer acetate, daclizumab, teriflunomide, fingolimod, dimethyl fumarate, alemtuzumab, mitoxantrone, ocrelizumab, natalizumab, prednisone, methylprednisolone, baclofen, tizanidine.

In particular, when the neurodegenerative disease is a Stroke, said second therapeutic agent can be an anti-clotting agent selected in the group consisting of anticoagulants (e.g. warfarin), antiplatelet (e.g. aspirin, dipyridamole, clopidogrel), or agents as tissue plasminogen activator, alteplase, statins, angiotensin II receptor blockers, ACE inhibitors, beta-blockers, calcium channel blockers, diuretics.

In particular, when the neurodegenerative disease is a Gaucher's disease, said second therapeutic agent can be an enzyme replacement treatment or a substrate reduction therapy selected in the group consisting of imiglucerase, velaglucerase, eliglustat, miglustat.

The invention also relates to a method of manufacturing a compound according to the invention, which comprises the step of selecting a compound which specifically binds to CD38 and which activates the opening of NAADP receptors Two Pore Channels TPC1 and/or TPC2.

In particular, the step of selecting an antibody or an antigen-binding fragment thereof or an antigen-binding antibody mimetic which specifically binds to CD38 according to the invention comprising or consisting of selecting an antibody or an antigen-binding fragment thereof or an antigen-binding antibody mimetic which has a KD value inferior or equal to 10⁻⁸ M, preferably inferior or equal to 10⁻⁹ M for CD38, more preferably inferior or equal to 1.10⁻¹⁰ M, as may be determined by biosensor analysis, particularly by Biacore Analysis.

In particular, the step of selecting a small organic molecule which specifically binds to CD38 according to the invention comprising or consisting of selecting a small organic molecule which has a KD value inferior or equal to 10⁻⁶ M, preferably inferior or equal to 10⁻⁷ M for CD38, more preferably inferior or equal to 1.10⁻⁸ M, as may be determined by biosensor analysis, particularly by Biacore Analysis.

In particular, the step of selecting an oligonucleotide which specifically binds to CD38 according to the invention comprising or consisting of selecting a small organic molecule which has a KD value inferior or equal to 200 nM, preferably inferior or equal to 150 nM for CD38, more preferably inferior or equal to 100 nM, as may be determined by biosensor analysis, particularly by Biacore Analysis.

As above indicated, the capacity of a compound to activate the opening of NAADP receptors Two Pore Channels TPC1 and/or TPC2 can be tested by using an inhibitor of TPC1 and/or TPC2 activation, such as NedK and Ned-19, as described in the examples below.

In particular, the step of selecting a compound which activates the opening of NAADP receptors Two Pore Channels TPC1 and/or TPC2 comprising or consisting of selecting a compound, which neuroprotective effect is antagonized by at least 20%, 30%, 40%, 50%, particularly at least 60% and more particularly at least 70% in the presence of Ned-19, as compared to a negative control in the absence of Ned-19 in a neuroprotective assay as described in the examples below. These neuroprotective assays include but are not limited to assays on prevention of spontaneous and progressive dopaminergic (DA) neuron death in midbrain cultures, protection of dopaminergic neurons against the mitochondrial neurotoxin MPP⁺ and protection of cortical neurons from excitotoxicity, in the presence or absence of Ned-19, as described in the examples below.

The method of the invention can further comprise the step of selecting a compound which inhibits the NAADP hydrolase activity of CD38 or which activates the NAADP synthase activity of CD38.

The method of the invention can further comprise the step of selecting a compound which inhibits the ability of CD38 to degrade NAADP or which activates the ability of CD38 to synthetize NAADP.

The capacity of a compound to inhibit the NAADP hydrolase activity of CD38 (the ability of CD38 to degrade NAADP) or to activate the NAADP synthase activity of CD38 (the ability of CD38 to synthetize NAADP) can be tested using methods described in Schmid et al. (2011, FEBS letters, vol. 585, Issue 22, 3544-3548) as mentioned above.

The method of the invention can further comprise the step of selecting a compound which increases intracellular NAADP levels, in particular in neurons, by inhibiting the ability of CD38 to degrade NAADP (by inhibiting the NAADP hydrolase activity of CD38) or by activating the ability of CD38 to synthetize NAADP (by activating the NAADP synthase activity of CD38), preferably by at least 10%, 20%, 30%, 40%, 50%, particularly at least 60% and more particularly at least 70%, as compared to a negative control molecule, in a NAADP level measurement assay.

The method of the invention can further comprise the step of selecting a compound which increase cytosolic calcium levels, in particular in neurons, preferably by at least 10%, 20%, particularly at least 30% and more particularly at least 40%, as compared to a negative control molecule, in an intracellular calcium level measurement assay.

The method of the invention can also comprise the step of selecting an antibody or antigen binding fragment thereof or an antigen-binding antibody mimetic which specifically binds to:
- at least one peptide consisting of amino acids 220 to 285 of human CD38; and/or
- to cysteine 254 and/or 275 of human CD38; and/or
- to the 5^{th} C-terminal disulphide loop involving cysteine 254 and 275 of human CD38.

The method of the invention can also comprise the step of selecting a small organic molecule which inhibits the ability of CD38, in particular human CD38, to degrade NAADP with an IC50 inferior or equal to 5 µM, in particular inferior or equal to 500 nM, more particularly inferior or equal to 50 nM or activates the ability of CD38, in particular human CD38, to synthetize NAADP with an EC50 inferior or equal to 5 µM, in particular inferior or equal to 500 nM, more particularly inferior or equal to 50 nM.

The method of the invention can also comprise the step of selecting a small organic molecule which specifically binds to at least one, in particular at least two, more particularly the three amino-acid(s) of human CD38 selected from the group consisting of glutamic acid 146, aspartic acid 155 and glutamic acid 226.

### BRIEF DESCRIPTION OF DRAWINGS

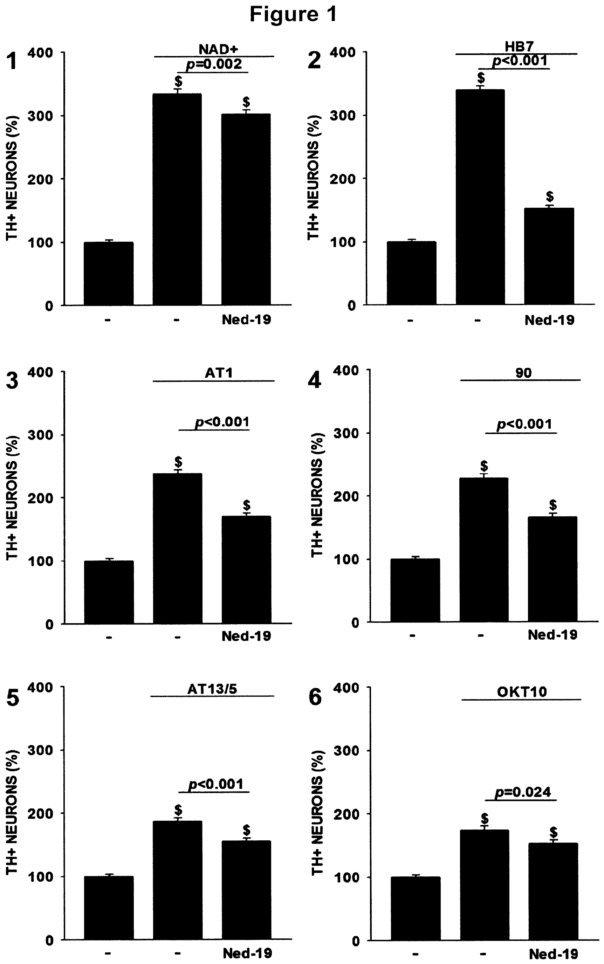

**Figure 1** is a bargraph showing the neuroprotective effect of NAD⁺ (**1**) and several anti-CD38 antibodies (HB7 (**2**), AT1 (**3**), clone 90 (**4**), AT13/5 (**5**) or OKT-10 (**6**)) in a model of midbrain cultures where these neurons degenerate spontaneously, selectively and progressively as they mature. Number of DA (TH⁺) neurons in 10-day in vitro (DIV) cultures chronically exposed to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. Results are expressed in % of TH⁺ neurons in control cultures at DIV10. $ P <0.05 vs untreated cultures.

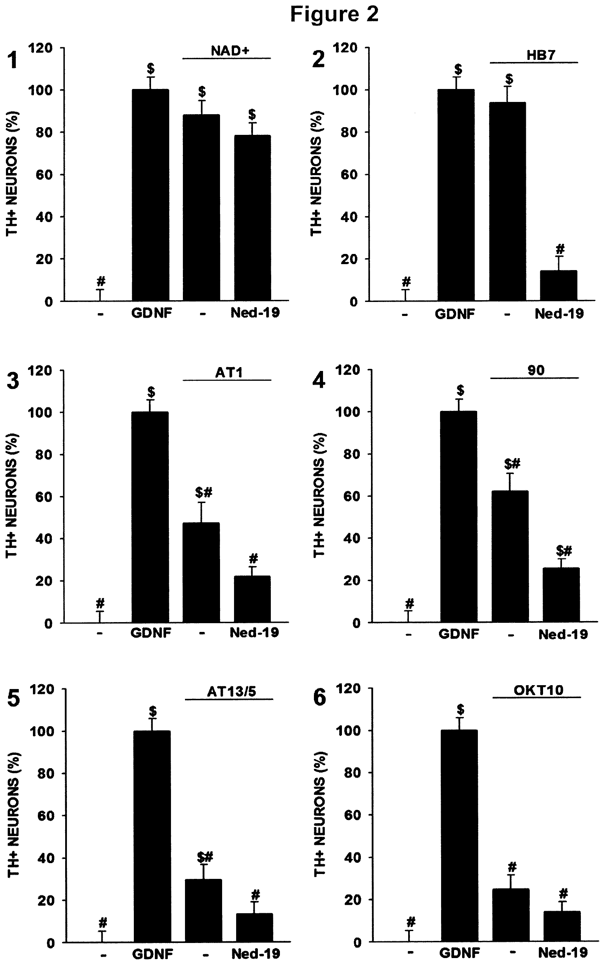

**Figure 2** is a bargraph showing the neuroprotective effect of NAD⁺ (**1**) and several anti-CD38 antibodies (HB7 (**2**), AT1 (**3**), clone 90 (**4**), AT13/5 (**5**) or OKT-10 (**6**)) for DA (TH+) neurons in a culture model where these neurons degenerate following GDNF withdrawal. DA cell survival in midbrain cultures initially exposed to 20 ng/ml GDNF for 10 DIV and then deprived of the peptide between 11 and 15 DIV exposed or not to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. GDNF was used as positive control at a concentration of 20 ng/ml. Results are expressed in % of neurons in the undeprived condition. $ P <0.05 vs untreated cultures; # P <0.05 vs cultures treated with GDNF.

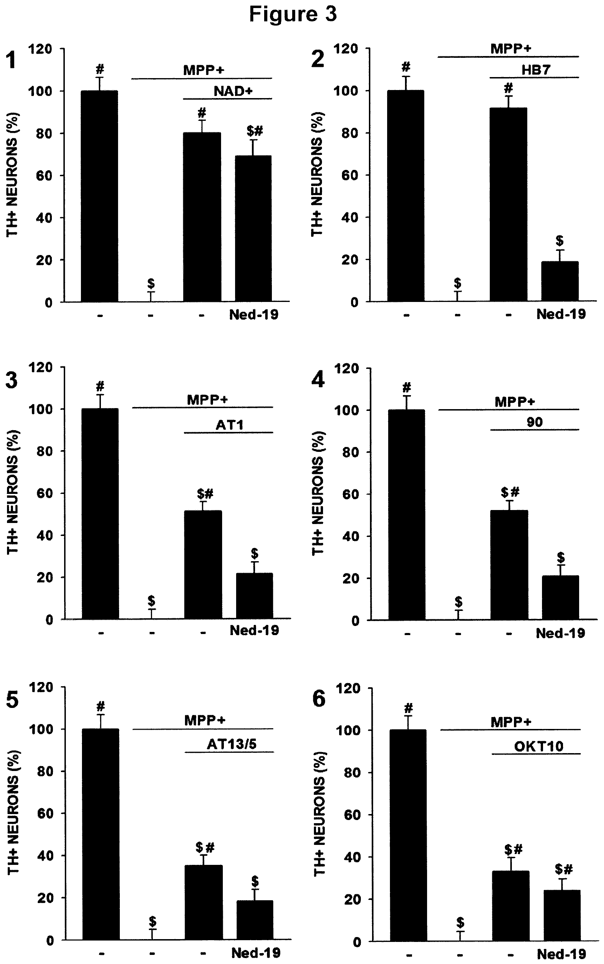

**Figure 3** is a bargraph showing the neuroprotective effect of NAD⁺ (**1**) and several anti-CD38 antibodies (HB7 (**2**), AT1 (**3**), clone 90 (**4**), AT13/5 (**5**) or OKT-10 (**6**)) against MPP⁺-induced DA cell death. DA (TH⁺) cell survival in midbrain cultures treated with MPP⁺ (3 µM) between 5 and 7 DIV exposed or not to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. Results are expressed in % of corresponding control cultures. $ P <0.05 vs control treatment; # P <0.05 vs MPP⁺ treatment.

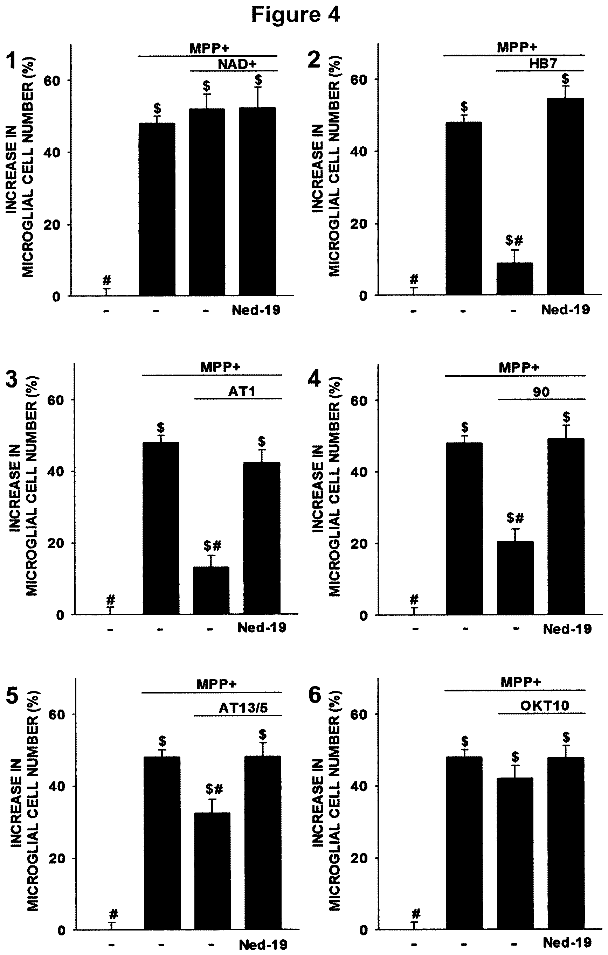

**Figure 4** is a bargraph showing the effect of NAD⁺ (**1**) and several anti-CD38 antibodies (HB7 (**2**), AT1 (**3**), clone 90 (**4**), AT13/5 (**5**) or OKT-10 (**6**)) on microglial cells following MPP⁺ (3 µM) treatment. Increase in the number of microglial cells in midbrain cultures treated with MPP⁺ (3 µM) between 5 and 7 DIV exposed or not to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. Results are expressed in % of corresponding control cultures. $ P <0.05 vs control treatment; # P <0.05 vs MPP⁺ treatment.

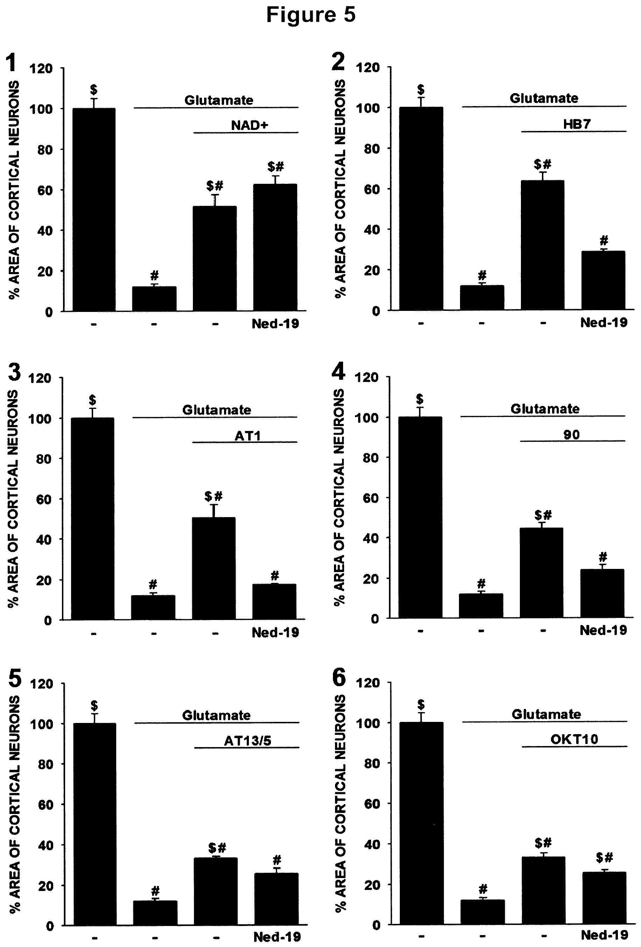

**Figure 5** is a bargraph showing the neuroprotective effect of NAD⁺ (1) and several anti-CD38 antibodies (HB7 (**2**), AT1 (**3**), clone 90 (**4**), AT13/5 (**5**) or OKT-10 (**6**)) against excitotoxicity triggered by a prolonged treatment of cortical cultures with glutamate (75 µM). Neuronal cell survival in cortical cultures treated with glutamate (75 µM) between 12 and 14 DIV and exposed to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. Results are expressed in % of corresponding control cultures. $ P <0.05 vs glutamate treatment; # P <0.05 vs control treatment.

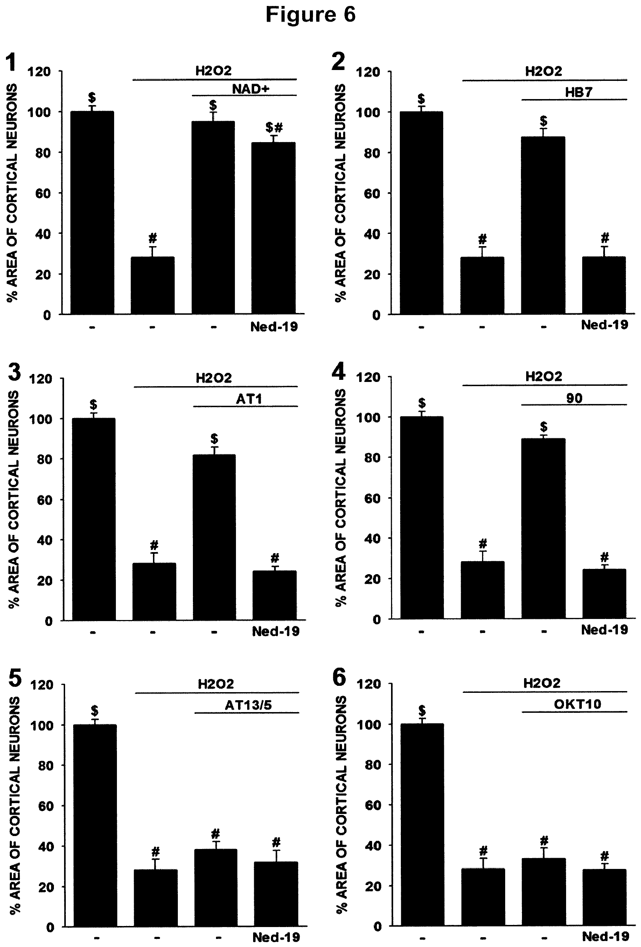

**Figure 6** is a bargraph showing the neuroprotective effect of NAD⁺ (**1**) and several anti-CD38 antibodies (HB7 (**2**), AT1 (**3**), clone 90 (**4**), AT13/5 (**5**) or OKT-10 (**6**)) against oxidative stress triggered by a treatment of cortical cultures with H₂O₂ (75 µM). Neuronal cell survival in cortical cultures treated with H₂O₂ (75 µM) between 12 and 14 DIV and exposed to NAD⁺ (3 mM) or several anti-CD38 antibodies (clone 90, AT1, AT13/5, OKT-10 or HB-7; 1 µg.ml) in the presence or not of Ned-19 (2 µM), an inhibitor of NAADP receptor activation. Results are expressed in % of corresponding control cultures. $ P <0.05 vs H₂O₂ treatment; # P <0.05 vs control treatment.

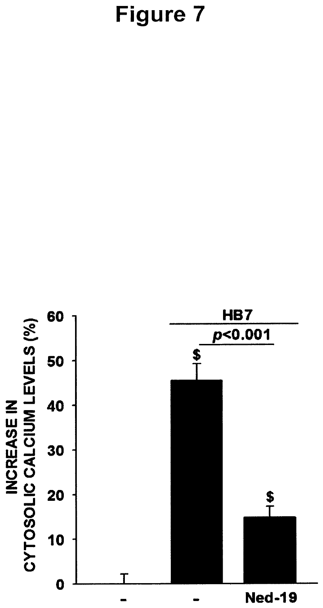

**Figure 7** is a bargraph showing the effect of HB7 antibody on cytosolic calcium levels in the presence or absence of Ned-19 (2µM). Increase in cytosolic calcium levels in cortical cultures between 7 and 10 DIV exposed or not to HB-7 (1 µg.ml) in the presence or not of Ned-19 (2 µM), an inhibitor of NAADP receptor activation. Results are expressed in % of corresponding control cultures. $ P <0.05 vs control treatment.

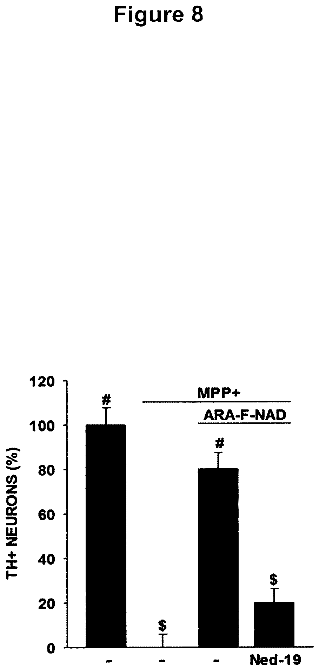

**Figure 8** is a bargraph showing the neuroprotective effect of ara-2'-F-NAD⁺ (ARA-F-NAD, 200µM) against MPP⁺-induced DA cell death. DA (TH⁺) cell survival in midbrain cultures treated with MPP⁺ (3 µM) between 5 and 7 DIV exposed or not to ARA-F-NAD in the presence or not of Ned-19 (2 µM), an inhibitor of NAADP receptor activation. Results are expressed in % of corresponding control cultures. $ P <0.05 vs control treatment; # P <0.05 vs MPP⁺ treatment.

The present invention is described in the following examples, but the technical scope of the present invention is not limited to these examples.

### EXAMPLES

**Table 1 summarizes information, in particular, the epitope binding sequence within CD38, of the antibodies used in the examples.**

| CD38 antibody Name | Immunogen species | Commercial reference | Epitope sequence binding (*) | Reference | Effect on CD38 NADase (glycohydrolase) activity | Effect on CD38 cyclase activity | Reference |
|---|---|---|---|---|---|---|---|
| Clone 90 | Mouse | BioLegend ref 102702 | unknown | | None on cell surface CD38 | Inhibit | Hara-Yokoyama (Internatio nal Immunoph armacolog y, 2008, 8, 59-70) |
| AT1 | Human | Santa Cruz Biotechnolog y ref sc-7325 | the 5^{th} C-terminal disulfide loop involving cysteine 254 and 275 and cysteines 254 and 275 | Ferrero (BMC Immunolog y, 2004, 5 :21) | unknown | unknown | |
| AT13/5 | Human | Santa Cruz Biotechnolog y ref sc-59028 | 273-300 | Ellis (J. Immunolog y 1995, 155:925-937) | unknown | none | Ellis (J. Immunolo gy 1995, 155:925-937) - Deckert (Clinic Cnacer Research, 2014, 20(17) |
| HB7 | Human | BioLegend ref 356602 | 220 to 285, and the 5^{th} C-terminal disulfide loop involving cysteine 254 and 275 and cysteines 254 and 275 | Hoshino (J. Immunolog y, 1997, 158:741-747) | none | none | Deckert (Clinic Cancer Research, 2014, 20(17)) Hoshino (J. Immunolo gy, 1997, 158:741-747) |
| | | | | Zhao (The Journal of Biological Chemistry, 2011, vol. 286) | | | |
| OKT10 | Human | Anticorps en ligne ABIN270425 8 | 285 to 300 and the 6^{th} C-terminal disulfide loop involving cysteine 287 and 296 | Ferrero (BMC Immunolog y, 2004, 5 :21) | unknown | none | Deckert (Clinic Cancer Research, 2014, 20(17):457 4-83 |

| | | | | | | | |
|---|---|---|---|---|---|---|---|
| (*)The numbering of amino acids of human CD38 consisting of said "epitope sequence binding" corresponds to the numbering of amino acids of the human CD38 sequence referenced by the NP_001766 NCBI accession number. | | | | | | | |

### Example 1: Neuroprotection of midbrain dopaminergic neurons by anti-CD38 antibodies

### Prevention of spontaneous and progressive dopaminergic (DA) cell death in midbrain cultures.

We report here that NAD⁺ (3 mM) and several anti-CD38 antibodies (clone 90, AT1, AT13/5, OKT-10 or HB-7; 1 µg/ml) increased the number of midbrain DA (TH⁺) neurons in culture conditions where these neurons die spontaneously, selectively and progressively as they mature (**Figure 1**). Noteworthy, the neuroprotective effect of anti-CD38 antibodies (clone 90, AT1, AT13/5, OKT-10 or HB-7), and slightly but significantly that of NAD⁺, was antagonized in the presence of Ned-19 (2 µM), an inhibitor of NAADP receptor activation.

### Protection of DA neurons that degenerate after GDNF deprivation.

To determine whether the protective effect of NAD⁺ (3 mM) and several anti-CD38 antibodies (clone 90, AT1, AT13/5, OKT-10 or HB-7; 1 µg/ml) remained observable in more mature DA neurons (**Figure 2**), we used midbrain cultures in which the spontaneous death process was postponed for 10 days by chronic application of GDNF (20 ng/ml). Ablation of GDNF from these cultures at 11 DIV led to a massive and selective loss of TH⁺ neurons within the next 5 days in agreement with previous data (Guerreiro et al., Mol Pharmacol. 2008 Oct;74(4):980-9). Interestingly, a large population of these neurons were saved from death by NAD⁺ (3 mM) or anti-CD38 antibodies (clone 90, AT1, AT13/5, or HB-7; 1 µg/ml) but not by clone OKT-10 anti-CD38 antibody (1 µg/ml). The neuroprotective effect of anti-CD38 antibodies clone 90, AT1, AT13/5, or HB-7 but not that of NAD⁺ was abolished in the presence of Ned-19 (2 µM), an inhibitor of NAADP receptor activation.

### Protection against the mitochondrial neurotoxin MPP⁺.

To determine whether NAD⁺ (3 mM) and several anti-CD38 antibodies (clone 90, AT1, AT13/5, OKT-10 or HB-7; 1 µg/ml) remained protective in a situation in which DA cell death was caused by mitochondrial poisoning with MPP⁺, the active metabolite of the DA neurotoxin MPTP was tested. For this purpose, spontaneously occurring DA cell death was prevented by a treatment combining depolarizing concentrations of K⁺ (30 mM) and MK801 (5 µM), a glutamate receptor antagonist used to prevent unwanted excitotoxic insult. The cultures were then exposed to 3 µM MPP⁺ between 5 and 7 DIV to achieve a loss of approximately 50% of DA neurons. When the cultures were exposed to NAD⁺ (3 mM) or anti-CD38 antibodies (clone 90, AT1, AT13/5, OKT-10 or HB-7; 1 µg/ml) during the intoxication period, MPP⁺-induced DA cell loss was either partially or almost totally prevented by these compounds (**Figure 3**). The neuroprotective effect of anti-CD38 antibodies clone 90, AT1, AT13/5, or HB-7 but not that of NAD⁺ or clone OKT-10 anti-CD38 antibody (1 µg/ml) was abolished in the presence of Ned-19 (2 µM), an inhibitor of NAADP receptor activation.

### Example 2: Anti-inflammatory effect of anti-CD38 antibodies

### Limitation of the number of Microglial Cells.

Neuro-inflammation has been repeatedly implicated in neurodegeneration and microglial cells, the resident innate immune cells in the brain, may play a key role in this process. *In vitro,* MPP⁺ was shown to induce an increase in microglial cells number (Henze et al. J Neurochem. 2005 95(4):1069-77). We tested whether NAD⁺ (3 mM) and several anti-CD38 antibodies (clone 90, AT1, AT13/5, OKT-10 or HB-7; 1 µg/ml) were able to reduce the increase in the number of microglial cells observed following MPP⁺ application *in vitro.* We observed that anti-CD38 antibodies (clone 90, AT1, AT13/5 or HB-7; 1µg/ml) but not NAD⁺ (3 mM) or clone OKT-10 anti-CD38 antibody (1 µg/ml) reduced the number of microglial cells in 7 DIV midbrain cultures (**Figure 4**) and that this effect was suppressed when Ned-19 (2 µM) was concomitantly added to the cultures.

### Example 3: Neuroprotection of cortical neurons by anti-CD38 antibodies

### Protection from excitotoxicity.

Excitotoxicity occurring through an over-activation of glutamate receptors is thought to play a key role in neurodegeneration (Lewerenz J and Maher, Front Neurosci, 2015, 9:469). To determine whether NAD⁺ (3 mM) and several anti-CD38 antibodies (clone 90, AT1, AT13/5, OKT-10 or HB-7; 1µg/ml) protected DA neurons from excitotoxic insults, we tested these compounds in a model in which neurodegeneration is induced following glutamate (75 µM) exposure. We observed that cultures exposed to NAD⁺ (3 mM) or anti-CD38 antibodies (clone 90, AT1, AT13/5, OKT-10 or HB-7; 1 µg/ml) were protected from excitotoxicity (**Figure 5**). The neuroprotective effect of anti-CD38 antibodies clone 90, AT1, and HB7 but not that of NAD⁺ or anti-CD38 antibodies clone AT13/5 or OKT-10 (1 µg/ml) was abolished in the presence of Ned-19 (2 µM), an inhibitor of NAADP receptor activation.

### Protection from oxidative stress.

To determine whether NAD⁺ (3 mM) and several anti-CD38 antibodies (clone 90, AT1, AT13/5, OKT-10 or HB-7; 1µg/ml) protected DA neurons from oxidative stress insults, we tested these compounds in a model in which neurodegeneration is induced following H₂O₂ (75 µM) exposure. We observed that cultures exposed to NAD⁺ (3 mM) or anti-CD38 antibodies (clone 90, AT1 or HB-7; 1 µg/ml) but not those exposed to anti-CD38 antibodies clone AT13/5 or OKT-10 (1 µg/ml) were protected from oxidative stress (**Figure 6**). The neuroprotective effect of anti-CD38 antibodies clone 90, AT1, and HB7 but not that of NAD⁺ was abolished in the presence of Ned-19 (2 µM), an inhibitor of NAADP receptor activation.

### Neuroprotection is associated with an increase in intracellular free calcium levels.

The NAADP receptors TPC1 and TPC2 are known to release calcium from lysosomal compartment, leading to an increase in cytoplasmic calcium (Pitt et al., J Physiol, 2016, 594(15):4171-9). To determine whether cytosolic calcium levels are increased following anti-CD38 clone HB7 (1 µg.ml) treatment through the recruitment of NAADP receptors, we measured cytosolic calcium levels of cortical neurons in the presence or not of HB7 and / or the NAADP receptor antagonist Ned-19 (2 µM) (**Figure 7**). We observed that anti-CD38 clone HB7 antibody increased cytosolic calcium levels, and that this effect is abrogated in the presence of the NAADP receptor antagonist Ned-19 (2 µM).

### Example 4: Neuroprotection of midbrain dopaminergic neurons by small organic molecule CD38 inhibitor

To determine whether small organic molecule inhibitor of CD38 enzymatic activity were neuroprotective, we tested the effect of ara-2'-F-NAD+ (ARA-F-NAD), a slow-binding and selective inhibitor of CD38 (Muller-Steffner et al., J Biol Chem, 1992, 267(14):9606-11; Berthelier et al., Biochem J, 1998, 330:1383-90), in the MPP⁺ in vitro model. When the cultures were exposed to ARA-F-NAD (200µM) during the intoxication period, MPP⁺-induced DA cell loss was almost totally prevented by this CD38 inhibitor (**Figure 8**). The neuroprotective effect induced by ARA-F-NAD was abolished in the presence of Ned-19 (2 µM), an inhibitor of TPC 1 and/or 2 activation. This experiment demonstrates that this small organic molecule CD38 inhibitor is able to activate the opening of NAADP receptors TPC1 and/or TPC2, leading to protection of neurons.

### Materials and methods

### Midbrain cell cultures

Animals were treated in accordance with the Guide for the Care and Use of Laboratory Animals (National Research Council, 1996), European Directive 86/609, and the guidelines of the local institutional animal care and use committee. Cultures were prepared from the ventral mesencephalon of gestational age 15.5 Wistar rat embryos (Janvier Breeding Center, Le Genest St Isle, France). Dissociated cells in suspension obtained by mechanical trituration of midbrain tissue pieces were seeded at a density of 1.2-1.5 10⁵ cells/ cm² onto tissue culture supports precoated with 1 mg/ml polyethylenimine diluted in borate buffer pH 8.3 as described (Michel et al., J Neurochem, 1997, 69(4):1499-507). The cultures were then maintained in N5 medium supplemented with 5 mM glucose, 5% horse serum, and 0.5% fetal calf serum, except for the first 3 days in vitro (DIV), when the concentration of fetal calf serum was 2.5% to favor initial maturation of the cultures (Guerreiro et al., Mol Pharmacol, 2008 74(4):980-9). They were fed daily by replacing 70% of the medium. Routinely, mesencephalic cultures were established on Nunc 24-well culture plates (Thermofischer Scientific, Rochester, NY). Note that these cultures contain tyrosine hydroxylase (TH)⁺ neurons that were exclusively dopaminergic (Traver et al., Mol Pharmacol, 2006, 70(1):30-40). TH⁺ neurons represented approximately 1-2% of the total number of neuronal cells present in these cultures. The evaluation of the survival of DA neurons was performed by counting cells immunopositive for TH as described (Toulorge et al., Faseb J, 2011, 25(8):2563-73).

### Culture systems used to model midbrain dopaminergic neuron cell death

### Spontaneous dopaminergic (DA) cell death model

We used a model system in which DA cell loss was spontaneous. The demise of DA neurons was progressive after plating to reach approximately 65-70%after 10 DIV (Guerreiro et al., Mol Pharmacol, 2008, 74(4):980-9).

### GDNF withdrawal model

To evaluate the efficacy of our compounds in a more mature population of DA neurons, we also used a variation of the previous model system. More precisely, spontaneous DA cell death was prevented up to 10 DIV by a chronic treatment with GDNF (20 ng/mL) to favor the maturation of healthy DA neurons. Then, DA cell death was triggered by total withdrawal of GDNF for the next 5 days.

### MPP⁺ intoxication model

Treatments with MPP⁺ were performed in cultures where the spontaneous death process was prevented by long-term exposure to depolarizing concentrations of K⁺ (30 mM), in the presence of the glutamate receptor antagonist MK801 (5 µM) to prevent unwanted excitotoxic insults as described previously (Douhou et al., J Neurochem, 2001, 78(1):163-74). Treatments with MPP⁺ and potential neuroprotective molecules were carried out between 5 and 7 days in vitro (DIV). The number of microglial cells was evaluated in this same model since MPP⁺ is known to induce microglial proliferation (Henze et al., J Neurochem, 2005, 95(4):1069-77).

### Cortical cell cultures

Animals were treated in accordance with the Guide for the Care and Use of Laboratory Animals (National Research Council, 1996), European Directive 86/609, and the guidelines of the local institutional animal care and use committee. Cultures were prepared from the cortex of gestational age 15.5 Wistar rat embryos (Janvier Breeding Center, Le Genest St Isle, France). Dissociated cells in suspension obtained by mechanical trituration of cortical tissue pieces were seeded onto tissue culture supports precoated with 1 mg/ml polyethylenimine diluted in borate buffer pH 8.3 according to the protocol previously described for mesencephalic cultures. The cultures were then maintained in N5 medium supplemented with 5 mM glucose, 5% horse serum, and 0.5% fetal calf serum, except for the first 3 days in vitro (DIV), when the concentration of fetal calf serum was 2.5% to favor initial maturation of the cultures (Guerreiro et al., Mol Pharmacol, 2008, 74(4):980-9). They were fed daily by replacing 70% of the medium. Routinely, cortical cultures were established on Nunc 24-well culture plates (Thermofischer Scientific, Rochester, NY).

### Culture system used to model cortical neuron cell death

### Excitotoxicity model

Glutamate (75 µM) was added to cortical cultures between 12 and 14 days in vitro (DIV) in the presence or the absence of potential neuroprotective treatments. The cultures were then fixed in formaldehyde and immunostained using anti-MAP-2 antibody. The area occupied by MAP-2⁺ neurons was assessed using ImageJ.

### Oxidative stress model

H₂O₂ (75 µM) was added to cortical cultures between 12 and 14 days in vitro (DIV) in the presence or the absence of potential neuroprotective molecules. The cultures were then fixed in formaldehyde and immunostained using anti-MAP-2 antibody. The area occupied by MAP-2⁺ neurons was assessed using ImageJ.

### Quantification of neuronal survival and microglial cells number

The cultures were fixed for 12 min using 4% formaldehyde in Dulbecco's phosphate-buffered saline (PBS), then washed twice with PBS before an incubation step at 4 °C for 24 h with the following antibodies. A monoclonal anti-TH antibody diluted 1/5000 (ImmunoStar, Inc., Hudson, WI) or a polyclonal anti-TH antibody diluted 1/1000 (US Biologicals, Salem, MA) was used to assess the survival of DA neurons. A monoclonal anti-MAP2 antibody diluted at 1/250 (clone AP20, Sigma-Aldrich) was used to assess the survival of cortical neurons. Microglial cells were characterized using a mouse anti-Iba1 antibody (1/50; clone MRC OX-42; Pharmingen, BD Biosciences, Le Pont-de-Claix, France). All antibodies were diluted in PBS containing 0.2% Triton X-100, except the mouse anti-lbal which was diluted in PBS only. Detection of the primary antibodies was performed with an Alexa Fluor-488 conjugate of an anti-mouse IgG antibody or with an Alexa Fluor-555 conjugate of an anti-rabbit antibody (1:500).

### Measurement of cytosolic Ca²⁺ levels

Cytoplasmic free calcium levels were measured in individual cortical neurons using Cal-520 (AAT Bioquest). In brief, cultures grown for 7 to 8 days were incubated with 10 µM Cal-520. for 30 min at 37°C, washed twice with serum-free glucose-supplemented N5 medium to remove excess indicator, and then left to recover in the presence of test compounds for 30 min before assessment. The fluorescent signal (excitation, 480 nm; emission, 510 nm) was quantified using the Simple-PCI software from C-Imaging Systems and a Nikon (Tokyo, Japan) TE-300 inverted microscope equipped with an ORCA-ER digital camera from Hamamatsu (Bridgewater, NJ). The average pixel intensity over the surface of each cell body was determined under the different experimental conditions. Background fluorescence was subtracted from raw data, and the results were expressed as a percentage of mean fluorescence intensity per cell under control conditions. A minimum of 180 cells were analysed under each test condition.

### Statistical Analysis

Simple comparisons between two groups were performed with Student's *t* test. Multiple comparisons against a single reference group were made by one-way analysis of variance followed by Dunnett's test when possible. When all pairwise comparisons were required, the Student-Newman-Keuls test was used. S.E.M. values were derived from at least three independent experiments.

## Claims

1. A compound which specifically binds to CD38, for use as a medicament in the prevention and/or treatment of a neurodegenerative disease, by the opening of NAADP receptors Two Pore Channels TPC1 and/or TPC2, wherein said compound activates the opening of NAADP receptors Two Pore Channels TPC1 and/or TPC2.

2. The compound according to claim 1 for use according to claim 1, wherein said compound is selected form the group consisting of:
- an antibody, an antigen-binding fragment thereof, an antigen-binding antibody mimetic; and
- a small organic molecule.

3. The compound according to claim 1 or 2 for use according to claim 1 or 2, wherein said compound increases intracellular NAADP levels in neurons, by inhibiting the NAADP hydrolase activity of CD38 or by activating the NAADP synthase activity of CD38.

4. The compound according to any one of claims 1 to 3 for use according to any one of claims 1 to 3, wherein said compound is an anti-CD38 antibody or an antigen binding fragment thereof or an antigen-binding antibody mimetic, which specifically binds to the peptide consisting of amino acids 220 to 285 of human CD38.

5. The compound according to any one of claims 1 to 4 for use according to any one of claims 1 to 4, wherein said compound is an anti-CD38 antibody or an antigen binding fragment thereof or an antigen-binding antibody mimetic, which specifically binds to cysteine 254 and/or 275 of human CD38.

6. The compound according to any one of claims 1 to 5 for use according to any one of claims 1 to 5, wherein said compound is an anti-CD38 antibody or an antigen binding fragment thereof or an antigen-binding antibody mimetic, which specifically binds to the 5^{th} C-terminal disulfide loop involving cysteine 254 and 275 of human CD38.

7. The compound according to any one of claims 1 to 6, for use according to any one of claims 1 to 6, wherein said compound is an anti-CD38 antibody or an antigen binding fragment thereof or an antigen-binding antibody mimetic, which specifically binds to human CD38 with a KD inferior or equal to 10⁻⁸ M, as may be determined by biosensor analysis.

8. The compound according to any one of claims 1 to 7, for use according to any one of claims 1 to 7, wherein said compound is a humanized monoclonal antibody.

9. The compound according to any one of claims 1 to 3 for use according to any one of claims 1 to 3, wherein said compound is a small organic molecule which inhibits the NAADP hydrolase activity of CD38 with an IC50 inferior or equal to 5 µM or activates the NAADP synthase activity of CD38 with an EC50 inferior or equal to 5 µM.

10. The compound according to any one of claims 1 to 3 or 9, for use according to any one of claims 1 to 3 or 9, wherein said compound is a small organic molecule, which specifically binds to at least one amino-acid of human CD38 selected from the group consisting of glutamic acid 146, aspartic acid 155 and glutamic acid 226.

11. The compound according to any one of claims 1 to 10, for use according to any one of claims 1 to 10, wherein the neurodegenerative disease is selected from the group consisting of parkinson's disease and related disorders including Parkinson's disease, Parkinson-dementia, autosomal recessive PARK2 and PARK6-linked Parkinsonism, atypical parkinsonian syndromes, including, progressive supranuclear palsy, corticobasal degeneration syndrome, Lewy bodies dementia, multiple system atrophy, Guadeloupean Parkinsonism and Lytigo-bodig disease; motor neuron diseases including amyotrophic lateral sclerosis, frontotemporal dementia, progressive bulbar palsy, pseudobulbar palsy, primary lateral sclerosis, progressive muscular atrophy, spinal muscular atrophy and post-polio syndrome; neuro-inflammatory diseases; Alzheimer's disease and related disorders including early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, Pick's disease, argyrophilic grain disease, posterior cortical atrophy, Wernicke-Korsakoff Syndrome; prion diseases; lysosomal storage diseases; leukodystrophies; Huntington's Disease; Down syndrome; spinal and bulbar muscular atrophy; stroke; HIV-Associated Neurocognitive Disorder; Tourette Syndrome; autosomal dominant spinocerebellar ataxia; Friedreich's Ataxia; Dentatorubral pallidoluysian atrophy; myotonic dystrophy; schizophrenia; age associated memory impairment; autism and autism spectrum disorders; attention-deficit hyperactivity disorder; chronic pain; alcohol-induced dementia; progressive non-fluent aphasia; semantic dementia; spastic paraplegia; fibromyalgia; post-Lyme disease; neuropathies; withdrawal symptoms; Alpers' disease; cerebro-oculo-facio-skeletal syndrome; Wilson's disease; Cockayne syndrome; Leigh's disease; neurodegeneration with brain iron accumulation; opsoclonus myoclonus syndrome; alpha-methylacyl-CoA racemase deficiency; Andermann syndrome; Arts syndrome; Marinesco-Sjögren syndrome; mitochondrial membrane protein-associated neurodegeneration; pantothenate kinase-associated neurodegeneration; polycystic lipomembranous osteodysplasia with sclerosing leukoencephalopathy; riboflavin transporter deficiency neuronopathy; and ataxia telangiectasia.

12. The compound according to claim 11, for use according to claim 11, wherein the neurodegenerative disease is selected from the group consisting of Parkinson's disease, Lewy body dementia, multiple system atrophy, Alzheimer's disease, progressive supranuclear palsy, corticobasal degeneration syndrome, frontotemporal dementia, amyotrophic lateral sclerosis, spinal and bulbar muscular atrophy, stroke, traumatic brain injuries, Huntington's disease, multiple sclerosis, Friedreich's ataxia, Charcot-Marie-Tooth disease, Creutzfeld-Jacob disease and other prion diseases, leukodistrophies, lysosomal storage diseases.

13. A combination product comprising as active ingredients:
- at least one compound as defined in any one of claims 1 to 10; and
- at least one second therapeutic agent selected from the group consisting of neuroprotective agents, symptomatic agents, probiotics and antibodies used to neutralize aggregated or aggregation-prone proteins,
for its use as a medicament in the prevention and/or treatment of a neurodegenerative disease, by the opening of NAADP receptors Two Pore Channels TPC1 and/or TPC2,
wherein said active ingredients are formulated for separate, simultaneous, or sequential administration.

14. A method of manufacturing a compound as defined in any one of claims 1 to 10, which comprises the step of selecting a compound which specifically binds to CD38 and which activates the opening of NAADP receptors Two Pore Channels TPC1 and/or TPC2.

15. The method according to claim 14, wherein said method further comprises the step of selecting a compound which inhibits the NAADP hydrolase activity of CD38 or which activates the NAADP synthase activity of CD38.
